(19) Europäisches Patentamt
European Patent Office
Office européen des brevets

(11) Veröffentlichungsnummer: **0 005 732**
**B1**

(12) # EUROPÄISCHE PATENTSCHRIFT

(45) Veröffentlichungstag der Patentschrift: **15.06.88**

(51) Int. Cl.$^4$: **A 61 K 31/55**

(21) Anmeldenummer: **79101298.2**

(22) Anmeldetag: **30.04.79**

(54) **Verwendung von 2-Amino-oxazolo- oder 2-Amino-thiazolo-5,4-drazepinen sowie deren Säureadditionssalzen zur Herstellung eines Arzneimittels für die Verwendung bei der Bekämpfung von Angina Pectoris.**

(30) Priorität: **12.05.78 DE 2820808**

(43) Veröffentlichungstag der Anmeldung:
**12.12.79 Patentblatt 79/25**

(45) Bekanntmachung des Hinweises auf die Patenterteilung:
**15.06.88 Patentblatt 88/24**

(84) Benannte Vertragsstaaten:
**BE CH DE FR GB IT LU NL SE**

(56) Entgegenhaltungen:
**BE-A- 771 330**

**Naunyn-Schmiedeberg's Arch. Pharmacol. Vol. 300 (1977) Seite 39 bis 46**
**Drugs of the future, Vol. III, Nr. 5 (1978) Seite 343-345**

**Journal of Pharmacology and Experimental Therapeutics, Vol. 219, Nr. 3 (1961) Seite 760-761**

**Kurzes Lehrbuch der Pharmacologie (1972) Georg thieme Verlag-Stuttgart S. 68**

(73) Patentinhaber: **Dr. Karl Thomae GmbH**
**Postfach 1755**
**D-7950 Biberach (Riss) (DE)**

(72) Erfinder: **Benedikter, Lothar, Dr.**
**Beim Forhäldele 13**
**D-7950 Biberach 1 (DE)**
Erfinder: **Kobinger, Walter, Prof. Dr.**
**Belghofergasse 27**
**A-1121 Wien (AT)**
Erfinder: **Pichler, Ludwig, Dr.**
**Gusshausstrasse 24/11**
**A-1040 Wien (AT)**
Erfinder: **Ihrig, Hanns, Dr.**
**Johann-Sebastian-Bach-Strasse 25**
**D-7950 Biberach 1 (DE)**
Erfinder: **Griss, Gerhart, Dr., Dipl.-Chem.**
**Schopperweg 1**
**D-7950 Biberach 1 (DE)**

(74) Vertreter: **Zumstein, Fritz jun., Dr. et al**
**Dr. F. Zumstein sen. Dr. E. Assmann Dr. R. Koenigsberger Dipl.-Ing. F. Klingseisen Dr. F. Zumstein jun. Bräuhausstrasse 4**
**D-8000 München 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**EP 0 005 732 B1**

**Beschreibung**

In der Belgischen Patentschrift 771 330 werden u. a. Azepin-Derivate der allgemeinen Formel

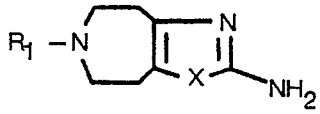

(I)

in der

$R_1$ ein Wasserstoffatom, eine gegebenenfalls durch eine Hydroxylgruppe substituierte Alkylgruppe mit 1 bis 4 Kohlenstoffatomen, eine Allylgruppe oder eine gegebenenfalls durch ein Halogenatom, eine Methyl-, Methoxy- oder Trifluormethylgruppe substituierte Benzylgruppe und

X ein Sauerstoff- oder Schwefelatom bedeuten und deren physiologisch verträgliche Säureadditionssalze mit anorganischen oder organischen Säuren beschrieben.

In dieser Patentschrift wird desweiteren festgestellt, daß die Verbindung der obigen allgemeinen Formel I wertvolle pharmakologische Eigenschaften aufweisen, je nach ihrer Substitution insbesondere eine blutdrucksenkende, sedative, hustenstillende und/oder antiphlogistische Wirkung. Die Azepin-Derivate der allgemeinen Formel I, in der $R_1$ eine Alkylgruppe mit 1 bis 4 Kohlenstoffatomen, eine Allylgruppe oder eine gegebenenfalls durch ein Halogenatom, eine Methyl-, Methoxy- oder Trifluormethylgruppe substituierte Benzylgruppe und X ein Schwefelatom bedeuten, weisen hierbei insbesondere eine blutdrucksenkende Wirkung und die Azepin-Derivate der allgemeinen Formel I, in der $R_1$ ein Wasserstoffatom, eine gegebenenfalls durch eine Hydroxylgruppe substituierte Alkylgruppe mit 1 bis 4 Kohlenstoffatomen oder eine Allylgruppe und X ein Sauerstoffatom bedeuten, insbesondere hustenstillende Eigenschaften auf.

Außerdem ist auf Naunyn-Schmiedeberg's Arch. Pharmacol. Vol. 300 (1977) Seite 39 bis 46, Drugs of the future, Vol. III, Nr. 5 (1978) Seite 343—345, Journal of Pharmacology and Experimental Therapeutics, Vol. 219, Nr. 3 (1961) Seite 760—761 und Kurzes Lehrbuch der Pharmakologie (1972) Georg Thieme Verlag-Stuttgart S. 68 hinzuweisen.

Es wurde nun gefunden, daß die anspruchsgemäßen Azepin-Derivate und deren physiologisch verträgliche Säureadditionssalze mit anorganischen oder organischen Säuren wertvolle antanginöse Eigenschaften aufweisen und somit zur Prophylaxe und Behandlung der Angina Pectoris geeignet sind.

Gegenstand der vorliegenden Anmeldung ist somit die Verwendung von 2-Amino-oxazolo- oder 2-Amino-thiazolo[5,4-d]azepinen der allgemeinen Formel

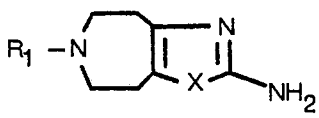

(I)

in der $R_1$ eine Alkylgruppe mit 1 bis 4 Kohlenstoffatomen oder die Allylgruppe und

X ein Sauerstoff- oder Schwefelatom bedeuten, sowie deren physiologisch verträgliche Säureadditionssalzen mit anorganischen oder organischen Säuren zur Herstellung eines Arzneimittels für die Verwendung bei der Bekämpfung von Angina Pectoris.

Die anspruchsgemäßen Azepin-Derivate können in dem Arzneimittel neben einem oder mehreren inerten Trägerstoffen und/oder Verdünnungsmitteln vorliegen. Sie werden in die üblichen pharmazeutischen Zubereitungen eingearbeitet. Die anspruchsgemäße Verwendung erfolgt zur Prophylaxe und Behandlung der Angina Pectoris. Die Einzeldosis beträgt hierbei 2 bis 10 mg, vorzugsweise 4 bis 7,5 mg, 1 bis 4 × täglich am Erwachsenen.

Besonders bevorzugte erfindungsgemäße Arzneimittel stellen jedoch diejenigen Zubereitungen dar, die als Wirksubstanz

2-Amino-6-äthyl-4,5,7,8-tetrahydro-6H-oxazolo[5,4-d]azepin und dessen Säureadditionssalze,

2-Amino-6-allyl-4,5,7,8-tetrahydro-6H-oxazolo[5,4-d]azepin und dessen Säureadditionssalze oder

2-Amino-6-allyl-4,5,7,8-tetrahydro-6H-thiazolo[5,4-d]azepin und dessen Säureadditionssalze enthalten.

Die antanginöse Wirksamkeit der Verbindung

A = 2-Amino-6-äthyl-4,5,7,8-tetrahydro-6H-oxazolo[5,4-d]azepin-dihydrochlorid

wurde an Angina pectoris Patienten wie folgt bestimmt:

Methodik:

11 Patienten mit klinischen und elektrokardiographischen Anzeichen einer Koronarinsuffizienz wurden für eine vergleichende, einfach blinde Prüfung der Verbindung A gegen Placebo ausgewählt. Die Auswahl erfolgte derart, daß nur solche Patienten in den Versuch genommen wurden, die bei 3 Ergometerbelastungen an 3 verschiedenen Tagen konstant bei der gleichen Wattzahl ihren präkordialen Schmerz berichteten. Am 4. Tag erhielten die Patienten Placebo in Form einer Injektion von physiologischer Kochsalzlösung, an den folgenden Tagen wurde dann die Testmedikation parenteral verabreicht. Die

Belastung der Patienten erfolgte sitzend auf dem Fahrradergometer und wurde eine halbe Stunde nach Injektion der Testsubstanz durchgeführt. Es wurde mit 25 Watt begonnen und stufenweise dreiminütlich um jeweils 25 Watt gesteigert. Herzfrequenz und Blutdruck wurden jede Minute registriert. Die Herzfrequenz wurde aus dem EKG ermittelt, der Blutdruck wurde manuell nach Riva-Rocci ständig von ein und derselben Medizinalassistentin gemessen. Die Belastung wurde gestoppt, wenn der Patient nicht mehr ertragbare präkordidale Schmerzen berichtete.

Als Beurteilungsparameter dienten:

1. Die Zeit bis zum Schmerzeintritt
2. Herzfrequenz
3. Blutdruck
4. Die geleistete Arbeit bis zum Abbruch der Belastung, die sich aus der Summe der Produkte von Zeit und minütlicher Wattzahl ergibt (W × min)
5. Die maximale Leistung, die sich aus der Wattzahl der letzten vollständigen dreiminütigen Belastungsperiode + der anteilmäßgen Wattzahl der nächsten abgebrochenen Periode ergibt (Watt)
6. Das Druckfrequenzprodukt als Index für den Sauerstoffverbrauch, das sich aus dem Produkt der Herzfrequenz und des systolischen Blutdrucks ergibt.

Da einige Patienten Herzschmerzen berichteten, ohne daß es hier-bei zu deutlichen ST-Senkungen kam, wurde der Eintritt und das Ausmaß der ST-Senkung nicht als Beurteilungsparameter herangezogen.

Ergebnis:

1. *Belastungsdauer*

Unter Kontrollbedingungen waren die Patienten im Mittel 6 Minuten belastbar, demgegenüber waren es nach Gabe von A im Durchschnitt 2 Minuten mehr.

2. *Herzfrequenz*

Bei Schmerzeintritt war die Frequenz nach Gabe von A um 2 Schläge/min erhöht, bei der höchsten vergleichbaren Belastung war die Herzfrequenz um 12 Schläge/min reduziert und bei der letzten vollständigen Minute vor Abbruch betrug die Differenz zwischen Placebo und Substanz A 3 Schläge/min.

3. *Blutdruck*

Bei Schmerzeintritt war der systolische Blutdruck nach Gabe von A um 8 mmHg reduziert. Der diastolische Blutdruck war nicht verändert. Zum Zeitpunkt der höchsten vergleichbaren Belastung war nach Gabe von A der systolische Blutdruck um 19 mmHg reduziert. Der diastolische Blutdruck bleibt wiederum unverändert. Bei der leitzten vollständigen Minute vor Abbruch war der systolische Blutdruck nach Gabe von A um 9 mmHg gegenüber Placebo reduziert.

4. *Arbeit bis zum Abbruch*

Unter Placebo leisteten die Patienten im Mittel eine Arbeit von 495 W × min und nach Gabe von A 627 W × min.

5. *Maximale Leistung*

Sie betrug unter Placebo 76 Watt und nach Gabe von A 87 Watt.

6. *Druckfrequenzprodukt*

Unter Placebo wurde ein Druckfrequenzprodukt von 310 und nach Gabe von A von 258 zum Zeitpunkt der höchsten vergleichbaren Belastung erreicht. Bei der letzten vollständigen Minute vor Abbruch betrug dieses Produkt Placebo 313 und nach Gabe von A 295.

Die Substanz A setzt die Herzfrequenz herab. Aus Tierversuchen ist bekannt, daß auch das Herzminutenvolumen herabgesetzt und das Schlagvolumen konstant bleibt. Die Herzarbeit wird hierdurch vermindert. Ferner läßt die Herabsetzung des Druckfrequenzprodukts auf eine Vermindung de Sauerstoffverbrachs und auf eine Ökonomisierung der Herzleistung und damit auf eine günstige Beeinflussung der Angina pectoris schließen.

Aus diesem Grunde wurde die Substanz A und die Verbindungen

B = 2-Amino-6-allyl-4,5,7,8-tetrahydro-6H-oxazolo[5,4-d]azepin-dihydrochlorid,
C = 2-Amino-6-äthyl-4,5,7,8-tetrahydro-6H-thiazolo[5,4-d]azepin-dihydrochlorid,
D = 2-Amino-6-n-propyl-4,5,7,8-tetrahydro-6H-thiazolo[5,4-d]azepin-dihydrochlorid,
E = 2-Amino-6-allyl-4,5,7,8-tetrahydro-6H-thiazolo[5,4-d]azepin-dihydrochlorid,

auf ihre herzfrequenzvermindernde Wirkung an Ratten, die vagotomiert wurden, wie folgt untersucht:

Die Tiere wurden vagotomiert und erhielten eine Trachealkanüle; in eine Jugularvene wurde ein Polyäthylen-Katheter eingebunden. Zur Registrierung des EKG's wurden an den Extremitäten Nadelelektroden angebracht. Während des Versuches wurde die Körpertemperatur der Tiere konstant gehalten (34°C).

Nach einstellen einer konstanten Ausgangsherzfrequenz (ermittelt durch Auszählen aus dem EKG) wurde dem Tier die Testsubstanz intravenös verabreicht. 30 Minuten später wurde neuerlich die Frequenz ermittelt.

Jede Substanz wurde in 3 Dosen an je 6 Tieren geprüft; je Tier lediglich eine Substanz und Dosis.

Die sechs Einzelwerte je Dosis (Δ Herzfrequenz in Schlägen/min) wurden gemittelt und die Dosiswirkungskurven aufgestellt; daraus wurden die $D_{50}$ (Dosis, die die Herzfrequenz um 50 Schläge/min. senkt) ermittelt:

| Substanz | $D_{50}$ in γ/kg i.v. |
|---|---|
| A | 500 |
| B | 100 |
| C | 32 |
| D | 12 |
| E | 42 |

Akute Toxizität:

Die akute Toxizität wurde an Mäusen beiderlei Geschlechts mit einem mittleren Gewicht von 20 g nach per os Applikation bestimmt. Aus dem Prozentsatz der Tiere, die nach den verschiedenen Dosen innerhalb von 14 Tagen verstarben, wurde nach Litchfield und Wilcoxon (J. Pharmacol. exp. Ther. *96*, 99 (1949)) die $LD_{50}$ berechnet:

| Substanz | $LD_{50}$ mg/kg p.o. |
|---|---|
| A | 2 210 |
| C | >1 500 |
| E | 455 |

Die Herstellung der Azepin-Derivate der allgemeinen Formel I wird in der Belgischen Patentschrift 771 330 beschrieben.

Die nachfolgenden Beispiele sollen die Erfindung näher erläutern:

Beispiel 1

Filmtabletten mit 5 mg 2-Amino-6-äthyl-4,5,7,8-tetrahydro-6H-oxazolo[5,4-d]azepin-dihydrochlorid

Zusammensetzung:

| | |
|---|---|
| Wirksubstanz | 5,0 mg |
| Milchzucker, direkt tablettierbar | 104,0 mg |
| Getrocknete Spezial-Maisstärke (STA—RX 1500) | 10,0 mg |
| Magnesiumstearat | 1,0 mg |
| | 120,0 mg |

Tablettenherstellung:

Die Tablettensestandteile werden gemischt und anschließend bei rel. Luftfeuchte ≤ 25% zu bikonvexen Tabletten verpreßt.

Tablettenmaße:

| | |
|---|---|
| Durchmesser: | 7 mm |
| Wölbungsradius: | 6 mm |
| Gewicht: | 120 mg |

Filmüberzug:

Die Tabletten werden im Dragierkessel mit einer $TiO_2$-haltigen äthanolischen Hydroxypropylmethylcellulose-Lösung (Pharmacoat) überzogen:

Filmgewicht: ca. 5 mg/Tablette.

Trocknung der Filmtabletten:

Die frisch gefertigten Filmtabletten werden in einem geeigneten Hochfrequenztrockner (27 bzw. 40 MHz, 8000 V Feldstärke) ≥ 5 min. lang getrocknet.

Verpackung:

Sie erfolgt in einem wasserdampfdichten Packmittel.

Beispiel 2

Filmtabletten mit 7,5 mg 2-Amino-6-äthyl-4,5,7,8-tetrahydro-6H-oxazolo[5,4-d]azepin-dihydrochlorid

Zusammensetzung:

| | |
|---|---|
| Wirksubstanz | 7,5 mg |
| Milchzucker, direkt tablettierbar | 101,5 mg |
| Getrocknete Spezial-Maisstärke (STA—RX 1500) | 10,0 mg |
| Magnesiumstearat | 1,0 mg |
| | 120,0 mg |

Herstellungsverfahren:

siehe Beispiel 1.

Beispiel 3

Ampullen mit 4 mg 2-Amino-6-äthyl-4,5,7,8-tetrahydro-6H-oxazolo[5,4-d]azepin-dihydrochlorid

Zusammensetzung:

| | | |
|---|---|---|
| Wirksubstanz | | 4,0 mg |
| Lactose | | 50,0 mg |
| 1 N NaOH ad pH | | 5,8 |
| ............ | | |
| Natriumchlorid | | 12,0 mg |
| Aqua dest. | ad | 2,0 ml |

Herstellungsverfahren:

Der Wirkstoff und Lactosemonohydrat werden in der Hauptmenge des Wasers für Injektionszwecke gelöst und mit 1 N Natronlauge auf einen pH-Wert von 5,8 eingestellt. Nach dem Auffüllen bis zur Eichmarke wird die Lösung uber ein Membranfilter filtriert und unter aseptischen Bedingungen in gereinigte und sterilisierte Injektionsfläschchen abgefüllt.

Der Inhalt wird in üblicher Weise lyophilisiert und die Fläschchen in einem Raum mit einer maximalen Luftefuchte von 10% zugebördelt.

Die Rekonstitution zur gebrauchsfertigen Injektionslösung erfolgt in einer in üblicher Weise hergestellten Nariumchloridlösung, deren Kochsalzgehalt so gewählt ist, daß die spritzfertige Lösung blutisotonisch wird.

Beispiel 4

Suppositorien mit 5 mg 2-Amino-6-äthyl-4,5,7,8-tetrahydro-6H-oxazolo[5,4-d]azepin-dihydrochlorid

Zusammensetzung:

| | |
|---|---|
| Wirksubstanz | 5,0 mg |
| Adeps solidus | 1 695,0 mg |
| | 1 700,0 mg |

Herstellungsverfahren:

Der Wirkstoff wird in der aufgeschmolzenen Suppositoriengrundlage mit Hilfe eines Rührers homogen verteilt. Diese homogene Schmelze wird direkt in vorgeformte Polyäthylen-beschichtete Alufolien ausgegossen.

**Patentansprüche**

1. Verwendung von 2-Amino-oxazolo- oder 2-Amino-thiazolo[5,4-d]azepinen der allgemeinen Formel

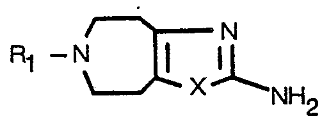

(I)

in der

$R_1$ eine Alkylgruppe mit 1 bis 4 Kohlenstoffatomen oder die Allylgruppe und

X ein Sauerstoff- oder Schwefelatom bedeuten, sowie deren physiologisch verträgliche Säureadditionssalzen mit anorganischen oder organischen Säuren zur Herstellung eines Arzneimittels für die Verwendung bei der Bekämpfung von Angina Pectoris.

2. Verwendung von 2-Amino-6-äthyl-4,5,7,8-tetrahydro-6H-oxazolo[5,4-d]azepin oder dessen physiologisch verträglichen Säureadditionssalzen gemäß Anspruch 1.

3. Verwendung von 2-Amino-6-allyl-4,5,7,8-tetrahydro-6H-oxazolo[5,4-d]azepin oder dessen physiologisch verträglichen Säureadditionssalzen gemäß Anspruch 1.

4. Verwendung von 2-Amino-6-allyl-4,5,7,8-tetrahydro-6H-thiazolo[5,4-d]azepin oder dessen physiologisch verträglichen Säureadditionssalzen gemäß Anspruch 1.

5. Verwendung gemäß einem der vorrstehenden Ansprüche, dadurch gekennzeichnet, daß das hergestellte Arzneimittel 2 bis 10 mg einer Verbindung der Formel I oder deren physiologisch verträglichen Säureadditionssalzen mit anorganischen oder organischen Säuren enthält.

**Revendications**

1. Utilisation de 2-amino-oxazolo- ou 2-aminothiazolo-[5,4-d]azépines de formule générale

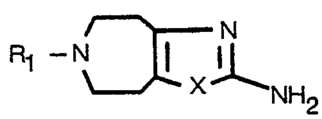

(I)

dans laquelle

$R^1$ représente un groupe alcoyle avec 1 à 4 atoms de carbone, ou le groupe allyle et

X représente un atome d'oxygène ou de soufre, ainsi que de leurs sels d'addition d'acides physiologiquement supportables avec des acides minéraux ou organiques, pour la préparation d'un médicament destiné à être utilisé pour combattre l'angine de poitrine.

2. Utilisation de la 2-amino-6-éthyl-4,5,7,8-tétrahydro-6H-oxazolo[5,4-d]azépine ou de ses sels d'addition d'acides physiologiquement supportables selon la revendication 1.

3. Utilisation de la 2-amino-6-allyl-4,5,7,8-tétrahydro-6H-oxazolo[5,4-d]azépine ou de ses sels d'addition d'acides physiologiquement supportables selon la revendication 1.

4. Utilisation de la 2-amino-6-allyl-4,5,7,8-tétrahydro-6H-thiazolo[5,4-d]azépine ou de ses sels d'addition d'acides physiologiquement supportables selon la revendication 1.

5. Utilisation selon l'une des revendications précédentes, caractérisée en ce que le médicament préparé contient 2 à 10 mg d'un composé de formule I ou de ses sels d'addition d'acides physiologiquement supportables avec des acides minéraux ou organiques.

**Claims**

1. The use of 2-amino-oxazolo- or 2-amino-thiazolo[5,4-d]azepines of the general formula

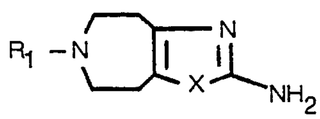

(I)

2

in which

$R_1$ is an alkyl group with 1 to 4 carbon atoms or allyl and

X is an oxygen atom or a sulfur atom, and the physiologically acceptable acid addition salts with inorganic or organic acids, in the production of a pharmaceutical preparation for use in combatting angina pectoris.

2. The use of 2-amino-6-ethyl-4,5,7,8-tetrahydro-6H-oxazolo[5,4-d]azepine or its physiologically acceptable acid addition salts according to claim 1.

3. The use of 2-amino-6-allyl-4,5,7,8-tetrahydro-6H-oxazolo[5,4-d]azepine or its physiologically acceptable acid addition salts according to claim 1.

4. The use of 2-amino-6-allyl-4,5,7,8-tetrahydro-6H-thiazolo[5,4-d]azepine or its physiologically acceptable acid addition salts according to claim 1.

5. The use according to any one of the foregoing claims, characterised in that the pharmaceutical composition contains 2 to 10 mg of a compound according to formula I or its physiologically acceptable acid addition salts with inorganic or organic acids.